# EUROPEAN PATENT APPLICATION

(11) **EP 1 844 838 A2**
(43) Date of publication of application: **17.10.2007**
(21) Application number: 07251319.5
(22) Date of filing: 28.03.2007
(51) Int. Cl.: B01D 46/44, B01D 46/46

(54) **Filter with memory, communication and temperature sensor**

(30) Priority: 12.04.2006 US 402737
(71) Applicant: MILLIPORE CORPORATION, Billerica Massachusetts 01821 (US)
(72) Inventor: Dileo, Anthony, Westford, MA 01886 (US)
(74) Representative: Kirkham, Nicholas Andrew

(57) **Abstract**

The present invention describes a system and method for accurately measuring the temperature of a filter element. A temperature transducer, and a communications device are coupled so as to be able to measure and transmit the temperature of a filter element while in use. This system can comprise a single component, integrating both the communication device and the temperature transducer. Alternatively, the system can comprise separate temperature transducer and transmitter components, in communication with one another. In yet another embodiment, a storage element can be added to the system, thereby allowing the device to store a set of temperature values. The use of this device is beneficial to many applications. For example, the ability to read filter temperatures in situ allows improved Sterilization-In-Place (SIP) protocol compliance, since the temperatures of actual filter elements can be directly measured, rather than interpolated as is done currently.

## Description

The use of RFID tags has become prevalent, especially in the management of assets, particularly those applications associated with inventory management. For example, the use of RFID tags permits the monitoring of the production line and the movement of assets or components through the supply chain.

To further illustrate this concept, a manufacturing entity may adhere RFID tags to components as they enter the production facility. These components are then inserted into the production flow, forming sub-assemblies in combination with other components, and finally resulting in a finished product. The use of RFID tags allows the personnel within the manufacturing entity to track the movement of the specific component throughout the manufacturing process. It also allows the entity to be able to identify the specific components that comprise any particular assembly or finished product.

In addition, the use of RFID tags has also been advocated within the drug and pharmaceutical industries. In February 2004, the United States Federal and Drug Administration issued a report advocating the use of RFID tags to label and monitor drugs. This is an attempt to provide pedigree and to limit the infiltration of counterfeit prescription drugs into the market and to consumers.

Since their introduction, RFID tags have been used in many applications, such as to identify and provide information for process control in filter products. U.S. Patent 5,674,381, issued to Den Dekker in 1997, discloses the use of "electronic labels" in conjunction with filtering apparatus and replaceable filter assemblies. Specifically, the patent discloses a filter having an electronic label that has a read/write memory and an associated filtering apparatus that has readout means responsive to the label. The electronic label is adapted to count and store the actual operating hours of the replaceable filter. The filtering apparatus is adapted to allow use or refusal of the filter, based on this real-time number. The patent also discloses that the electronic label can be used to store identification information about the replaceable filter.

A patent application by Baker et al, published in 2005 as U.S. Patent Application Publication No. US2005/0205658, discloses a process equipment tracking system. This system includes the use of RFID tags in conjunction with process equipment. The RFID tag is described as capable of storing "at least one trackable event". These trackable events are enumerated as cleaning dates, and batch process dates. The publication also discloses an RFID reader that is connectable to a PC or an internet, where a process equipment database exists. This database contains multiple trackable events and can supply information useful in determining "a service life of the process equipment based on the accumulated data". The application includes the use of this type of system with a variety of process equipment, such as valves, pumps, filters, and ultraviolet lamps.

Another patent application, filed by Jornitz et al and published in 2004 as U.S. Patent Application Publication No. 2004/0256328, discloses a device and method for monitoring the integrity of filtering installations. This publication describes the use of filters containing an onboard memory chip and communications device, in conjunction with a filter housing. The filter housing is equipped with a communication reader that is directly coupled to an integrity test instrument. (Alternately, the reader can be a hand held reader with its own memory for storing the data and which can be connected to an independent integrity test instrument). That application also discloses a set of steps to be used to insure the integrity of the filtering elements used in multi-round housings. These steps include querying the memory element to verify the type of filter that is being used, its limit data, and its production release data.

Despite the improvements that have occurred through the use of RFID tags, there are additional areas that have not been satisfactorily addressed. For example, in many applications, such as sterilization-in-place (SIP), the filter temperature, as measured while the filter element is in use, is an important consideration. Currently, it is not possible to reliably and accurately measure the filter temperature during the sterilization process in real time on the filter. To address this, sub-optimal configurations are used. For example, typically a temperature sensor is placed at a location distant from the filter element, and the filter temperature is interpolated from this sensor reading. While RFID tags offer one embodiment of the present invention, solutions utilizing wired communication are also envisioned. **SUMMARY OF THE INVENTION**

The shortcomings of the prior art are overcome by the present invention, which describes a system and method for accurately measuring the temperature of a filter element. In certain embodiments, a temperature transducer, and a communications device are coupled so as to be able to measure and transmit the temperature of a filter element, while in use. This system can comprise a single component, integrating both the communication device and the temperature transducer. Alternatively, the system can comprise separate temperature transducer and transmitter components, in communication with one another. In yet another embodiment, a storage element can be added to the system, thereby allowing the device to store a set of temperature values. In one embodiment, the communication device is able to wirelessly transmit information to the user. In a second embodiment, the communication device transmits the information via a wired connection to a point, typically outside the housing.

The use of this device is beneficial to many applications. For example, the ability to read filter temperatures in situ allows improved Sterilization-In-Place (SIP) protocol compliance, since the temperatures of actual filter elements can be directly measured, rather than interpolated as is done currently.

### BRIEF DESCRIPTION OF THE DRAWINGS

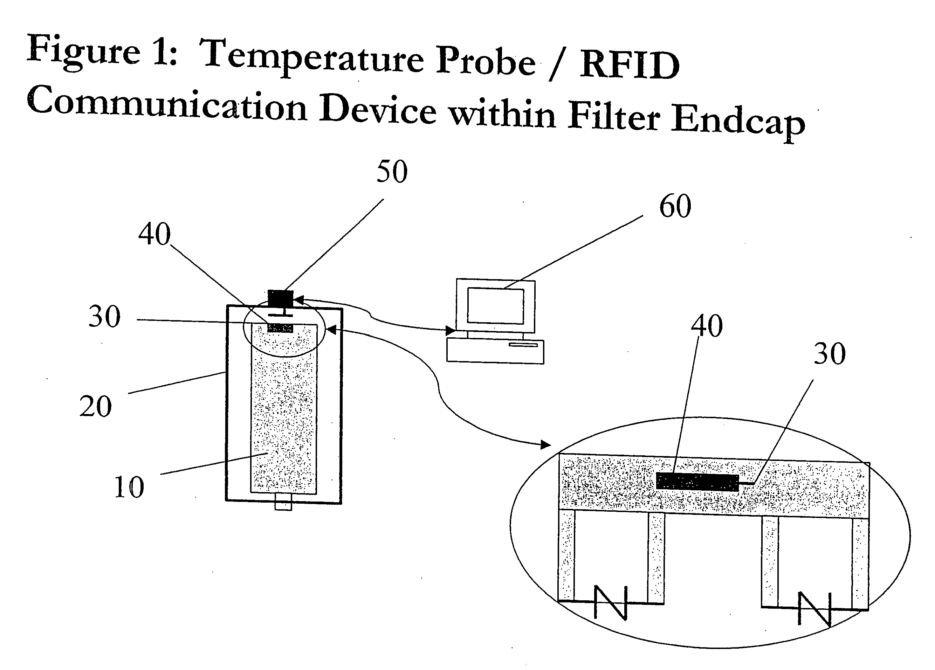

Figure 1 is a representative embodiment of the present invention.

### DETAILED DESCRIPTION OF THE INVENTION

Figure 1 illustrates a representative filtering system in accordance with the present invention. The filter element 10 is enclosed with a housing 20. The filter element can be simply a porous material, such as pleated paper or PVDF (Polyvinylidene fluoride) membrane. Alternatively, the filter element may comprise a frame, such as of plastic, and a porous material. Located in close proximity of, and preferably embedded in, the filter element 10 is a temperature sensor 30. This sensor 30 is capable of generating an output, which varies as a function of the surrounding temperature. This output can be in the form of an analog voltage or current, or can be a digital value. In the preferred embodiment, the output varies linearly with temperature, however this is not a requirement. Any output having a known relationship, such as logarithmic or exponential, to the surrounding temperature, can be employed. In such a situation, a transformation of the output can be performed to determine the actual measured temperature.

In one embodiment, the temperature sensor 30 is embedded in the end cap of the filter element 10. In other embodiments, the temperature sensor is affixed to, or embedded in, the filter element at a different point, preferably on the downstream side. In some applications, the temperature of the filter element may exceed 145°C, therefore a sensor capable of monitoring this temperature should be employed. Similarly, the temperature with the housing 20 may cycle from lower temperatures to higher temperatures and back, therefore the temperature sensor should have a response time sufficient to be able to measure temperature cycling. Suitable sensors include a thermistor, which is a resistor with a high temperature coefficient of resistance, and a transducer, which is an integrated circuit. The sensor can also be of another type, including, but not limited to, a diode, a RTD (resistance temperature detector) or a thermocouple.

In one embodiment, a wireless transmitter 40 is also located near, or integrated with, the temperature sensor 30. In the preferred embodiment, the wireless transmitter 40 and the temperature sensor 30 are encapsulated in a single integrated component. Alternatively, the transmitter 40 and the sensor 30 can be separated, and in communication with each other, such as via electrical signals. Various types of wireless communication devices are possible, although the use of an RFID tag is preferred. An active RFID tag allows regular communication with the reader, thereby obtaining the temperature profile continuously over time. Alternatively, a passive RFID tag can be used, whereby the energy to transmit and sense the temperature is obtained from the electromagnetic field transmitted by the RFID reader, thereby obtaining the temperature at a specific point in time corresponding to when the RFID element is activated by the reader. In some applications, the temperature of the filter element may exceed 145°C for up to one hour, therefore a transmitter capable of withstanding this temperature should be employed. Similarly, the temperature with the housing 20 may cycle from lower temperatures to higher temperatures and back, therefore the temperature sensor should be able to withstand temperature cycling. Mechanisms for transmitting wireless signals outside the housing have been disclosed. United States Patent Application Publication 2004/0256328 describes the use of an antenna to relay information between transponders located on the filter housing to a monitoring and test unit external to the housing.

Alternatively, the temperature sensor may be used in conjunction with a wired transmitter. In this embodiment, one or more wires, or other suitable conduits, are used to transmit the information from the temperature sensor to a location external to the filter housing.

Optionally, a storage element 50 can be used in conjunction with the wireless transmitter 40 and the temperature sensor 30. This storage element 50, which is preferably a random access memory (RAM), FLASH EPROM or NVRAM device, can be used to store a set of temperature readings, such as may be generated by regular sampling of the sensor. This allows the rate at which the wireless transmitter 40 sends data to be different from the rate at which the temperature is sampled. For example, the temperature may be sampled 10 times per second, while the data is transmitted only once per second. Similarly, the storage element must be capable of withstanding temperatures of 145°C for extended periods of time.

In the embodiment employing a wireless transmitter, a wireless receiver, 60, located outside the filter housing 20, is used to communicate with the transmitter. In the preferred embodiment, an RFID reader or base station is used. The reader can be configured such that it queries the transmitter at regular intervals. Alternatively, the reader can be manually operated so that readings are made when requested by the equipment operator. In another embodiment, the wireless receiver 60 also includes a storage element. This reduces the complexity required of the device within the housing. In this embodiment, the wireless receiver queries the wireless transmitter/temperature sensor at preferably regular intervals. It receives from the wireless transmitter the current temperature sensor measurement as determined at that time. The wireless receiver 60 then stores this value in its storage element. The capacity of the storage element can vary, and can be determined based on a variety of factors. These include, but are not limited to, the rate at which measurements are received, the rate at which the stored data is processed, and the frequency with which this storage element is in communication with its outside environment.

As an example, consider a filter element having a wireless transmitter 40, such as an RFID tag, coupled with a temperature sensor 30. In this embodiment, the RFID tag is passive, that is, it only sends data upon receipt of a query from the wireless receiver, or base station. Upon receipt of that query, the transmitter transmits the value currently available from the temperature sensor 30. In one scenario, the wireless receiver, which is coupled to a computing device, such as a computer, then stores these temperature values, optionally with an associated timestamp, such as in a log file. In a different scenario, if the wireless receiver is separated from the computer, the receiver will need to store a number of temperature measurements internally, until such time as it is connected to the main computing and/or storage device. In this case, a storage element needs to be integrated with the receiver.

Having defined the physical structure of the present invention, there are a number of applications in which it is beneficial. The following is meant to illustrate some of those applications, however it is not intended as a recitation of all such applications.

In one embodiment, the present invention is used in conjunction with sterilization using Steam-In-Place (SIP). SIP is a requirement mandated by the FDA, to insure adequate cleanliness of manufacturing equipment in accordance with cGMP. In this process, steam is introduced into the filter housing. This process requires that the operator certify that sterilization temperatures reach at least a minimum temperature. Conventionally, to insure compliance with this, the temperature was monitored on the outside of the housing at a "cold spot", and assumed to be at least that value for all of the filter elements contained within. Once this "cold spot" reached the required minimum temperature, the timing can begin. Typically, sterilization cycles last roughly 30 minutes. This method requires that the sterilization necessarily be performed at temperatures in excess of those required since the temperature of the filter element cannot be directly measured. A complete description of this process can be found a technical brief by Millipore Corporation, entitled "Steam-In-Place Method for Millipore Express SHF Filters", which is hereby incorporated by reference, as well as a technology primer, entitled "Principles of Steam-In-Place" by Jean-Marc Cappia, which is also hereby incorporated by reference.

The Sterilization using Steam-In-Place (SIP) can be performed more accurately and efficiently through the use of the present invention. The filter elements, composed of plastic, will heat more slowly than the stainless steel housing. Therefore, there is potential that the filter element may not be at the SIP temperature at the same time as the monitored cold spot. In this case, the temperatures of the various filter elements can be measured using the devices mounted directly on, or embedded in, the filters, minimizing error. In one embodiment, the temperature sensor will measure the temperature of the end cap of the filter, which will represent the temperature of the plastic in the filter element. Alternately, the sensor can be located at the junction of the membrane and the end cap. Correlations can be obtained between that temperature and the temperature within the filter pleats. Depending on the type of temperature sensor used, provision may be made for the calibration of the sensor. Given this capability to measure the temperature at the filter element, the validation of the SIP protocols will no longer be necessary.

A second application that benefits from this invention is monitoring temperatures within the filter housing adjacent to the filter element during pressure decay integrity testing. In these tests, gas is pumped into the housing until it reaches a certain pressure. The pressure decay is then monitored as the gas diffuses through the filter elements. If the pressure drops too quickly, it is assumed that the gas flow is no longer via diffusion, but rather via convection. Determination of the point at which this transition occurs is critical in an integrity test.

These integrity tests are performed assuming that the temperature remains constant throughout the test, or is a specific value throughout the test, or changes at a constant rate that is significantly smaller than the measured pressure decay. However, these assumptions are typically untrue. According to the ideal gas law, expressed as (PV = nRT), as the gas is pressurized within the housing, its temperature will necessarily increase since the volume is held constant. Thus, since the temperature varies from that assumed value, either in absolute value or if the change in temperature with time is comparable to the change in pressure with time, the result achieved may be erroneous. The ability to monitor and measure the temperature, especially the instantaneous change in temperature with time, within the housing during these various integrity tests can alleviate this problem in several ways. First, it insures that test results are valid, in that it can verify that the temperature within the housing was as required. Second, an algorithm utilizing the ideal gas law can account for temperature and temperature changes explicitly. This algorithm can therefore remove temperature effects from the interpretation of the measurement to obtain a corrected and more accurate estimate of the test results. Third, since the actual temperature can be measured, the tests can be executed more quickly since it is no longer necessary to wait a predetermined amount of time for the temperature within the housing to stabilize or decay to a certain value, which is currently the only action that can be taken to eliminate temperature effects in a pressure decay integrity test measurement.

In one embodiment, a plastic filter housing is utilized, allowing the wireless transmitter to transmit pressure data through the housing at any time.

## Claims

1. An apparatus for monitoring the temperature of a filtering element, comprising:
said filtering element,
a temperature sensor in close proximity to said
filtering element, and
a transmitter, in communication with said
temperature sensor.

2. The apparatus of claim 1, further comprising a storage element in communication with said temperature sensor adapted to store measurements from said temperature sensor.

3. The apparatus of claim 1 or 2, wherein said temperature sensor is selected from the group consisting of a thermistor, a thermocouple, a temperature transducer, diode and a resistance thermal detector.

4. The apparatus of any one of claims 1 to 3, wherein the transmitter comprises a wireless transmitter.

5. The apparatus of claim 4, wherein said wireless transmitter comprises an RFID tag.

6. The apparatus of any one of claims 1 to 5, wherein the temperature sensor and said transmitter are provided in a single enclosure.

7. The apparatus of any one of the preceding claims, further comprising a wireless receiver, adapted to receive signals transmitted from said transmitter.

8. The apparatus of any one of the preceding claims, wherein the temperature sensor is embedded in said filtering element.

9. The apparatus of claim 8, wherein:(a) said filtering element comprises an end cap, and said temperature sensor is embedded in said end cap; or (b) said temperature is embedded in the downstream side of said filtering element.

10. A method of insuring the validity of a Sterilization using Steam-In-Place procedure performed on a filtering element within a filter housing, comprising:
providing a temperature sensor, in communication with a transmitter, in close proximity to said filtering element;
performing said sterilization-in-place procedure;
monitoring the temperature of said filtering element during said procedure; and
verifying that said monitored filter temperature is
greater than a predefined minimum temperature.

11. The method of claim 10, wherein said monitoring step is performed at regular intervals.

12. The method of claim 10 or 11, wherein said transmitter comprises a wireless transmitter, and said method further comprises the step of wireless transmitting said monitored temperature to a receiver located outside of said housing.

13. The method of any one of claims 10-12, wherein said temperature sensor is embedded in said filtering element.

14. A method for removing temperature variations from integrity test measurements utilizing pressurized gas, performed on a filtering element within a filter housing, comprising:
providing a temperature sensor, in communication with a transmitter, in close proximity to said filtering element;
performing said integrity test procedure;
monitoring the temperature of said gas within the housing during said procedure; and
incorporating said monitored temperature measurement
into the interpretation of said integrity test.

15. The method of claim 14, wherein said incorporating step either comprises rejecting the results of said test if said monitored temperature is not within a predetermined range; and/or
comprises adjusting the results of said test in response to said monitored temperature.

16. The method of claim 14 or 15, further comprising monitoring the pressure of said gas, and normalizing said monitored pressure based on said monitored temperature.

17. The method of any one of claims 14 to 16, wherein said temperature sensor is embedded in said filtering element.
